# EUROPÄISCHE PATENTSCHRIFT

(11) **EP 0 575 999 B1**
(45) Veröffentlichungstag und Bekanntmachung des Hinweises auf die Patenterteilung: **22.09.1999**
(21) Anmeldenummer: 93110070.5
(22) Anmeldetag: 24.06.1993
(51) Int. Cl.: G01N 33/53, G01N 33/74

(54) **Verfahren zum Nachweis eines Analyten enthaltend glykosidische Tenside**
Process for the detection of an analyte containing glycoside detergents
Procédé de décèlement d'un analyte contenant des détergents glycosidiques

(30) Priorität: 26.06.1992 DE 4220653
(43) Veröffentlichungstag der Anmeldung: 29.12.1993
(73) Patentinhaber: Roche Diagnostics GmbH, 68298 Mannheim (DE)
(72) Erfinder: Sluka, Peter, Dr., D-8120 Weilheim (DE); Wehner, Rainer, Dr., D-8132 Tutzing (DE)

(56) Entgegenhaltungen:
- EP-A- 0 496 409
- WO-A-89/08843
- CHEMICAL ABSTRACTS, Band 112, Nr. 13, 26 März 1990, Columbus, Ohio, USA Y. MIZUMURA et al. "Plates for detection of antibodies to Newcastle disease virus and octyl glucoside solubilization of virus antigen." Seite 407, Zusammenfassung-Nr. 115 384b & JP-A-01 245 159
- BIOCHEMISTRY, Band 19, Juli-September 1980, American Chemical Society, New York P. ROSEVEAR et al. "Alkyl Glycoside Detergents: A Simpler Syntesis and Their Effects on Kinetic and Physical Properies of Cytochrome c Oxidase." Seiten 4108-15

## Beschreibung

Die Erfindung betrifft ein heterogenes Verfahren zum Nachweis eines Analyten, bei dem man den Analyten mit mindestens einem spezifischen Rezeptor zusammenbringt, der an eine Festphase gebunden ist oder gebunden werden kann, indem man dem Inkubationsmedium ein wasserlösliches glykosidisches Tensid zusetzt.

Verfahren zum Nachweis eines Analyten in einer Probe, wobei man den Analyten mit einem spezifischen Rezeptor zusammenbringt, der an eine Festphase gebunden ist oder gebunden werden kann, sind bekannt. Sie werden allgemein als heterogene Verfahren bezeichnet. Im Falle von Immunoassays sind verschiedene Verfahrensvarianten bekannt, z. B. das Sandwich-Verfahren, das indirekte Verfahren und das Kompetitionsverfahren.

Beim Sandwich-Verfahren wird ein Antikörper an den Träger gebunden, die Testlösung wird zugegeben, wobei das in der Testlösung enthaltene spezifische Antigen an den Antikörper gebunden wird. Dann wird ein markierter spezifischer Antikörper für den Antigen-Antikörper-Komplex oder für Teile dieses Komplexes zugegeben, der an diesen Komplex bindet. Über den markierten Antikörper kann dann die Menge an Antigen berechnet werden.

Beim indirekten Verfahren wird ein Antigen an dem Trägermaterial gebunden. Man gibt Testlösungen dazu, wobei der in der Testlösung enthaltene, für das trägergebundene Antigen spezifische Antikörper mit dem Antigen reagiert. Bei Zugabe eines markierten Antiglobulins bindet das Antiglobulin an den Antigen-Antikörper-Komplex und die Menge des unbekannten Antikörpers in der Testlösung kann bestimmt werden.

Bei dem Kompetitionsverfahren wird einer der Partner der Immunoreaktion an das Trägermaterial gebunden. Man gibt dann eine Lösung dazu, die sowohl den in unbekannter Menge vorliegenden anderen Partner der Immunoreaktion enthält, als auch eine bekannte Menge an markiertem anderen Partner der Immunoreaktion. Beide Partner, der markierte und der unmarkierte, konkurrieren um die Bindungsstelle des am Träger gebundenen Partners der Immunoreaktion.

In allen diesen Verfahrensvarianten braucht der wandgebundene Rezeptor nicht direkt an die Festphase gebunden zu sein. Es ist möglich, einen wandgebundenen zweiten Rezeptor zu nutzen, der spezifisch mit dem ersten Rezeptor bindet und diesen im Laufe der Testführung, wobei ein oder mehrstufige Testführungen möglich sind, an die Festphase zu binden.

Im Falle, daß der Analyt eine Nukleinsäure ist, handelt es sich bei dem Verfahren um einen Hybridisierungstest, bei dem ein Rezeptor, in diesem Fall eine zu der nachzuweisenden Nukleinsäure komplementäre Nukleinsäure, an die Festphase gebunden ist oder gebunden werden kann. Auch hier sind dem Fachmann unterschiedliche Verfahrensvarianten bekannt.

Alle diese bekannten heterogenen Verfahren zum Nachweis eines Analyten gebrauchen meist Zusätze von Proteinen, Polysacchariden und/oder Tensiden, die nicht an der spezifischen Reaktion beteiligt sind, die jedoch das Ergebnis des heterogenen Verfahrens günstig beeinflussen. Heterogene Verfahren werden in unterschiedlichem Ausmaß durch nicht analytspezifische Interferenzen, sogenannte unspezifische Störungen, verfälscht, die in der Fachwelt als "Matrix-effekt", "Background" oder "unspezifische Bindung" bezeichnet werden.

In der DE-A 36 38 767 wurden festphasenimmunochemische Tests beschrieben, denen Lactoferrin, foetales Kälberserum, Polyoxiethylen-20-Sorbitanmonolaureat (Tween ® 20) zugesetzt wird. Durch den Zusatz von Tween ® 20 im Inkubationsmedium in Konzentrationen, die höher sind als 0.01 %, kann der an das Trägermaterial gebundene Rezeptor abgelöst werden, was wiederum zu einer negativen Beeinflussung des Nachweisverfahrens führt.

In der EP-A-0 215 457 wurden daher zur Vermeidung unspezifischer Störungen bei Immunobestimmungen in heterogener Phase nicht-ionische blockcopolymere Tenside mit einem HLB-Wert über 20, wie z. B. Tetronic ® eingesetzt. Diese Tenside haben den Vorteil, daß sie im Vergleich zu Tween ® 20 nicht so stark zur Desorption des wandgebundenen Rezeptors führen. Diese blockcopolymere Tenside besitzen keine homogene Zusammensetzung sondern weisen eine mehr oder weniger breite Homologenverteilung auf. Die Zusammensetzung ist einer chargenabhängigen Schwankung unterworfen. Daher muß jede Charge auf ihre Eignung in dem entsprechenden Nachweisverfahren zur Vermeidung unspezifischer Störungen jeweils neu geprüft werden. In der Vergangenheit zeigte sich dabei, daß einige Chargen nicht eingesetzt werden konnten.

Ein weiterer Nachteil der bisher gebräuchlichen Tenside liegt darin, daß zu deren Herstellung giftige und cancerogene Ausgansstoffe wie beispielsweise Ethylenoxid zum Einsatz kommen. Tenside vom Typ der Ethylen-Propylenoxid-Blockcopolymere sind außerdem biologisch schwer abbaubar.

Weniger giftige Tenside wie beispielsweise glykosidische Tenside sind bereits seit langem bekannt. Bisher wurden diese Tenside jedoch nur zur Probenvorbereitung verwendet und anschließend sorgfältigst durch Dialyse oder Verdünnen wieder entfernt.

In der japanischen Patentanmeldung Nr. 01-245,159 wird ein ELISA-Test zum Nachweis von Antikörpern gegen Newcastle Disease Virus beschrieben, bei dem die Virus-Partikel mit dem Tensid Octylglykosid lysiert und anschließend die Microtiterplatte mit dem Lysat beschichtet werden. Das Tensid wird jedoch nur zur Lyse der Viruspartikel verwendet und durch anschließende Dialyse entfernt, so daß in der Inkubationslösung zur Durchführung des ELISA-Tests kein Tensid mehr vorhanden ist. Ein zusätzlicher Wasch-Schritt nach Beschichtung der ELISA-Platten zeigt, daß überschüssiges Tensid keinesfalls als spezifitätsfördernd angesehen wird. Eine mögliche entstörende Wirkung durch Tenside im Test wird nicht offenbart.

In der EP-A-0 496 409, die nach dem Prioritätstag dieser Patentanmeldung veröffentlicht wurde, wird ein enzymatisches, nicht-immunologisches Nachweisverfahren für gram-negative Bakterien beschrieben, bei dem zur Lyse von störenden gram-positiven Bakterien ein wasserlösliches glykosidisches Tensid zugesetzt wird. Anschließend wird die Bakterien-haltige Lösung auf ein Filter gegeben, um die lysierten störenden gram-positiven Bakterien und andere Störsubstanzen von den zu analysierenden gram-negativen Bakterien abzutrennen. Die gram-negativen Baktereien werden somit auf dem Filter immobilisiert. Bei der Passage durch den Filter wird das Tensid der Lyse-Lösung wieder entfernt. Während des enzymatischen Nachweises der Dehydrogenase-Aktivität der gram-negativen Bakterien ist das Tensid nicht mehr präsent.

In der internationalen Patentanmeldung WO 89/08843 wird ein Verfahren zur Herstellung eines Antigens beschrieben. Zur Aufreinigung des Antigens wird Octylglykosid als Tensid eigesetzt. Das Tensid wird ausschließlich zur Ablösung des Antigens von der Membran der Wirtszelle verwendet und durch anschließende Dialyse vollständig wieder entfernt. Das Tensid ist also während eines Immunoassays nicht im Inkubationsmedium enthalten.

Das Problem der unspezifischen Bindungen in immunologischen Nachweisverfahren ist bisher im Stand der Technik nicht zufriedenstellend gelöst worden.

Es bestand daher die Aufgabe, ein Verfahren zum Nachweis eines Analyten sowie ein hierfür geeignetes Mittel zu finden, bei dem unspezifische Bindungen verhindert werden. Das hierzu erforderliche Reagenz soll keinen oder nur unwesentlichen chargenabhängigen Schwankungen unterliegen und umweltverträglich sein.

Gelöst wurde die Aufgabe durch ein Verfahren zum Nachweis eines Analyten, bei dem man den Analyten mit mindestens einem spezifischen Rezeptor zusammenbringt, der an eine Festphase gebunden ist oder gebunden werden kann, das dadurch gekennzeichnet ist, daß man dem Inkubationsmedium ein wasserlösliches glykosidisches Tensid zusetzt.

Weiterer Gegenstand der Erfindung ist ein Mittel zum Nachweis eines Analyten, das mindestens einen spezifischen Rezeptor enthält, der an eine Festphase gebunden ist oder gebunden werden kann, das weiterhin ein wasserlösliches glykosidisches Tensid enthält.

Oberflächenaktive glykosidische Tenside sind als Waschmittelrohstoffe bereits seit über 50 Jahren bekannt, beispielsweise aus der österreichischen Patentschrift 1 35 333, in der die Herstellung von Laurylglucosid beschrieben ist. In der DE-A-37 23 826 wird ein Verfahren zur Herstellung von Alkylpolyglykosiden (APGs) beschrieben. Solche APGs sind kommerziell erhältlich beispielsweise unter dem Handelsnamen Plantaren ®. Die Synthese der glykosidischen Tenside erfolgt auf dem Weg der Transglucosidierung über ein Alkylglucosid. Bei den bekannten Synthesen von glykosidischen Tensiden können genau definierte Ausgangsprodukte eingesetzt werden, die zu gut definierten Produkten führen.

Eine chargenabhängige Schwankung, wie im Falle der Blockcopolymere zu beobachten ist, tritt hierbei nicht in einem störenden Ausmaß auf.

Ebenfalls geeignet sind die definierten glykosidischen Tenside wie beispielsweise Octyl-D-glucopyranosid, die über eine aufwendigere Synthese gemäß Biochemistry 19, 4108-4115 (1980) herstellbar sind. Im Unterschied zu den APGs, bei denen es sich um Mono-, Di- und Trisaccharide handelt, stellen diese Verbindungen echte Monosubstanzen dar.

Erfindungsgemäß werden im folgenden unter glykosidischen Tensiden Reaktionsprodukte aus Zuckern und Fettalkoholen verstanden, wobei als Zuckerkomponente Aldosen beziehungsweise Ketosen, beispielsweise Glucose, Fructose, Mannose, Galactose, Xylose oder Ribose in Betracht kommen. Besonders bevorzugt ist die Glucose.

Als Fettalkohole kommen sowohl aliphatische als auch aromatische Alkohole in Betracht. Dieser Fettalkohol muß derart ausgewählt werden, daß die glykosidischen Tenside noch gut wasserlöslich sind. Bevorzugt werden als aliphatische Alkohole solche verwendet, deren Alkylrest 4 - 18 Kohlenstoffatome besitzt, besonders bevorzugt sind Alkylreste mit 6 - 8 Kohlenstoffatomen.

Als aromatische Alkohole werden bevorzugt solche verwendet, die einen aromatischen Ring enthalten, an dem gegebenenfalls weitere wasserlöslich machende Gruppen wie -OH, -OCH₃ oder -SO₂ substituiert sind. Beispiele für aromatische Alkohole sind Benzylalkohol, Salicylalkohol und β-Phenyl-ethanol. Besonders geeignet ist Benzylalkohol.

Als besonders geeignete glykosidische Tenside haben sich Hexylglucosid, Octylglucosid und Benzylglucosid oder Gemische hiervon erwiesen.

Durch den Zusatz dieser glykosidischen Tenside werden unerwünschte Reaktionen bei Verfahren zum Nachweis von Analyten, bei denen mindestens ein spezifischer Rezeptor an eine Festphase gebunden ist oder gebunden werden kann, unterdrückt und gleichzeitig keine Desorption des an die Festphase gebundenen spezifischen Rezeptors bewirkt. Darüberhinaus unterliegen glykosidische Tenside keiner ausgeprägten Chargenschwankung, so daß von Charge zu Charge vergleichbare Ergebnisse erzielt werden.

Mit dem heterogenen Verfahren können alle bekannten Analyten wie Haptene, Antigene, Proteine, Antikörper oder Nukleinsäuren nachgewiesen werden. Bei der Probe, die den nachzuweisenden Analyten enthält, kann es sich beispielsweise um Blut, Serum, Plasma, Sekrete, Liquor, Urin oder Produkten aus Geweben handeln. Besonders ist das Verfahren zum Nachweis eines Analyten in Plasma und Serum geeignet, da hierbei sehr gut übereinstimmende Ergebnisse erzielt werden. In Plasma werden ohne den Einsatz der erfindungsgemäßen Tenside sehr häufig starke unerwünschte Nebenreaktionen beobachtet, so daß die Ergebnisse zwischen Plasmaund Serumproben sehr stark schwanken können.

Das erfindungsgemäße Verfahren ist auf alle Plasmaarten anwendbar. So kann es sowohl für Plasma, das zur Stabilisierung mit EDTA versetzt wurde, als auch für Plasma, das mit Heparin oder Citrat versetzt wurde, verwendet werden.

Das Verfahren kann als kompetitives, indirektes oder Sandwich-Verfahren durchgeführt werden. Je nach der Verfahrensvariante ist als spezifischer Rezeptor ein Hapten oder Antigen, wie im Falle des kompetitiven oder indirekten Verfahrens, ein Antikörper oder ein Fragment hiervor, wie im Falle eines Sandwich-Verfahrens oder eine Nukleinsäure beziehungsweise ein Nukleinsäurefragment, wie im Falle eines Hybridisierungstestes, zu verstehen.

Der spezifische Rezeptor kann dabei direkt an die Festphase gebunden sein oder im Verlaufe des Verfahrens an die Festphase gebunden werden. Für den Fall, daß der spezifische Rezeptor erst im Verlaufe des Verfahrens gebunden wird, besteht dieser bevorzugt aus einem Konjugat eines spezifischen Rezeptors, beispielsweise eines Antigens, Antikörpers oder eine Nukleinsäure und einer Substanz S1. An der Festphase ist dann eine mit der Substanz S1 spezifisch bindefähige Substanz S2 gebunden. Als spezifisch bindefähige Substanzpaare S1-S2 sind besonders Antigen-Antikörper, Hapten-Antikörper, Biotin-Avidin beziehungsweise Streptavidin, Protein-Antiprotein, Protein A-Immunoglobulin, Hämoglobin-Haptoglobin oder Enzym-Substrat geeignet. Bevorzugt wird als S1 Biotin und als S2 Streptavidin bzw. Avidin verwendet.

Als Festphase ist im Sinne der Erfindung ein wasserunlöslicher Träger, an dem ein oder mehrere spezifische Rezeptoren gebunden sind oder gebunden werden können, zu verwenden.

Festphasen sind beispielsweise Latexpartikel, Kugeln, Röhrchen und Mikrotiterplatten aus verschiedenen Kunststoffmaterialien wie beispielsweise Polystyrol aber auch saugfähige oder poröse Materialen. Solche Festphasen sind dem Fachmann an sich bekannt.

Auch die Art und Weise der Markierung, die letztendlich zum Nachweis des an die Festphase gebundenen Komplexes aus spezifischem Rezeptor und Analyt genutzt wird, ist dem Fachmann bekannt.

Es können alle üblichen Markierungsmittel, wie radioaktive Marker, Enzyme, fluoreszierende oder chemilumineszierende Substanzen, zur Anwendung gelangen.

Der Zusatz der wasserlöslichen glykosidischen Tenside kann bei jeder für die Bestimmung verwendeten Lösungen erfolgen. Die glykosidischen Tenside können auch bereits bei der Vorbereitung der Probe für den Test zugefügt werden. Bevorzugt werden die Tenside dem Reaktionspuffer, bei einem zwei-oder mehrstufigen Verfahren, entsprechend den Reaktions-puffern, zugesetzt. Die Puffer können neben den Puffersubstanzen und den gegebenenfalls darin enthaltenden spezifischen Rezeptoren oder markierten Rezeptoren weiterhin stabilisierende Zusätze wie Proteine oder Polysaccharide, Konservierungsmittel und andere übliche Zusatzstoffe enthalten.

Die wasserlöslichen glykosidischen Tenside werden in einer Menge von 0,1 bis 2 % bezogen auf das Gewicht des Gesamtreaktionsansatzes eingesetzt. Bevorzugt ist eine Konzentration von 0,5 bis 1,0 %.

Zur Durchführung des Verfahrens wird ein Mittel verwendet, das einem spezifischen Rezeptor, der an eine Festphase gebunden ist oder gebunden werden kann, enthält und dadurch gekennzeichnet ist, daß es ein wasserlösliches glykosidisches Tensid enthält. Dieses Mittel kann außerdem noch an sich übliche Inhaltsstoffe enthalten. Beispielsweise können Puffer, Proteine wie Rinderserumalbumin oder IgG und/ oder Konservierungsmittel enthalten sein. Außerdem enthält es noch ein Konjugat aus einer Markierung und einem spezifischen Rezeptor oder im Falle des kompetitiven Tests ein Konjugat aus Markierung und Analyt beziehungsweise Analytanalogen. Wird als Markierung ein Enzym verwendet, so enthält das Mittel noch ein System zum Nachweis der Enzymaktivität.

Bevorzugt enthält das Mittel als glykosidische Tenside APGs auf der Basis von Hexylglucosid, Octylglucosid oder Benzylglucosid oder Gemische hiervon oder die analogen definierten Monosubstanzen. Die glykosidischen Tenside sind in dem Mittel in einer Konzentration von 0,1 - 2 % bezogen auf das Gewicht des Gesamtreaktionsansatzes enthalten.

Mit dem erfindungsgemäßen Verfahren gelingt es, die Wiederfindung bei Analyt-Bestimmungen in heterogener Phase zu verbessern. Darüberhinaus werden unerwünschte Nebenreaktionen unterdrückt, wie insbesondere die unspezifische Anlagerung von Konjugaten an die feste Phase, ohne daß es zu einer verstärkten Ablösung der gebundenen Reaktionspartner kommt. Die Probleme bei der Nutzung von Proben, die unerwünschte Nebenreaktionen zeigen, vor allem Plasmaproben, werden beseitigt. Durch die definiertere und homogenere Zusammensetzung der APGs, bzw. durch die definierte Zusammensetzung der glykosidischen Tenside hergestellt gemäß Biochemisty 19 (1980), 4108-4115 werden Schwankungen zwischen verschiedenen Chargen minimiert. Ein weiterer positiver Effekt ist, daß die glykosidischen Tenside eine gute bis sehr gute Umweltverträglichkeit zeigen.

Die Erfindung wird durch folgende Beispiele verdeutlicht:

### Beispiel 1:

### Herstellung von Hexyl-glucopyranosid

400 g 1-Hexanol wird zusammen mit 3,6 g p-Toluolsulfonsäure in einem 2 l Mehrhalskolben mit Rührer und Destillations-aufsatz vorgelegt und auf 110 °C erwärmt. Dann wird eine Suspension von 300 g wasserfreier Glucose in 300 g 1-Hexanol portionsweise, und zwar in 4 Portionen, zum Ansatz gegeben. Nach Zugabe der 1. Portion wird Vakuum (300 mbar) angelegt und das entstehende Reaktionswasser zügig abdestilliert. Sobald sich alles gelöst hat, wird die nächste Portion zugegeben. Mit den übrigen Portionen wird analog verfahren. Sobald nach der Zugabe der letzten Portion nur noch Hexanol übergeht, wird der Ansatz belüftet und mit Natriumethylat auf pH 8 gestellt. Das Hexanol wird anschließend bei 80 °C im Hochvakuum abdestilliert. Der ölige Rückstand wird in 7 l Wasser gelöst und 2 x mit je 1 l Essigester extrahiert. Die Wasserphase wird eingeengt und anschließend lyophilisiert. Man erhält 275 g einer schwach gelblich gefärbten Substanz.

### Beispiel 2:

### Herstellung von Benzyl-glucopyranosid

800 g Benzylalkohol wird zusammen mit 3,6 g p-Toluolsulfonsäure in einem 3 l Mehrhalskolben mit Rührer und Destillationsaufsatz vorgelegt und auf 110 °C erwärmt. Dann wird eine Suspension von 300 g wasserfreier Glucose in 500 g Benzylalkohol portionsweise, und war in 4 Portionen, zum Ansatz gegeben. Nach Zugabe der 1. Portion wird Vakuum angelegt und das entstehende Reaktionswasser zügig abdestilliert. Die nächsten Portionen werden jeweils dann zugegeben, wenn sich die Glucose klar gelöst hat. Sobald nach der Zugabe der letzten Portion nur noch Benzylalkohol übergeht, wird der Ansatz belüftet und der Katalysator mit Natriumethylat neutralisiert. Der Benzylalkohol wird anschließend bei 80 °C im Hochvakuum abdestilliert. Der ölige Rückstand wird in 2 l Wasser gelöst. Nach 8 Stunden scheidet sich der überschüssige Benzylalkohol am Gefäßboden ab. Die wässrige Phase wird dekantiert und 1 x mit 400 ml Essigester extrahiert. Die Wasserphase wird weiter eingeengt und anschliessend lyophilisiert. Man erhält 360 g einer schwach gelblich gefärbten Festsubstanz.

### Beispiel 3:

Am Beispiel des Enzymun-Testes ® LH der Firma Boehringer Mannheim GmbH wurde der Einfluß verschiedener Detergenzien auf den Eichkurvenverlauf ermittelt. Der Test zum Nachweis des luteinisierenden Hormons (LH) wurde entsprechend den Angaben des Herstellers durchgeführt.

Dem Inkubationspuffer (40 mmol/l Phosphat-Puffer pH 7,4) wurde Tween ® 20 bzw. Hexylglucosid oder Benzylglucopyranosid in Konzentrationen zwischen 0,005 und 1 % zugesetzt.

Tabelle 1 zeigt den Einfluß von Tween ® 20 auf den Eichkurvenverlauf des Enzymun-Test ® LH. Bei einer Konzentration von 0,5 % Tween ® 20 verläuft die Eichkurve um 60 - 70 % flacher im Vergleich zur Eichkurve ohne Detergenszusatz.

Tabelle 2 zeigt den Einfluß des erfindungsgemäßen Zusatzes der glykosdischen Detergenzien Hexylglucosid und Benzyl-glucopyranosid auf den Eichkurvenverlauf. Bei einem Zusatz von 0.5 % Detergens weist die Eichkurve lediglich eine Abflachung von 5 - 10 % im Vergleich zur Kontrolle ohne Detergenszusatz auf.

**Tabelle 1**

| **Enzymun-Test ® LH** | | | | |
|---|---|---|---|---|
| **Einfluß Tween ® 20 auf Eichkurvenverlauf** | | | | |
| Gemessen wurde die Extinktion in mE bei 405 nm. Als Probe dienten die Standards a - f (LH in Rinderserummatrix) des Enzymun-Test ® LH | | | | |

| Tween ® 20 Konzentration in % | 0 | 0,005 | 0,05 | 0,5 |
|---|---|---|---|---|
| Standard a (mE) | 29 | 21 | 20 | 23 |
| Standard b (mE) | 68 | 56 | 41 | 38 |
| Standard c (mE) | 208 | 167 | 111 | 85 |
| Standard d (mE) | 491 | 386 | 272 | 165 |
| Standard e (mE) | 1208 | 982 | 665 | 405 |
| Standard f (mE) | 2117 | 1677 | 1198 | 766 |

**Tabelle 2**

| **Enzymun-Test ® LH** | | | |
|---|---|---|---|
| **Einfluß glykosidischer Detergenzien auf Eichkurvenverlauf** | | | |
| Gemessen wurde die Extinktion bei 405 nm | | | |
| | Konzentration (%) | standard a (mE) | standard f (mE) |
| ohne Detergens | - | 25 | 2045 |
| Hexylglucosid | 0.1 | 21 | 1986 |
| Hexylglucosid | 0.5 | 13 | 1837 |
| Hexylglucosid | 1 | 12 | 1615 |
| Benzylglucopyranosid | 0.1 | 18 | 1979 |
| Benzylglucopyranosid | 0.5 | 19 | 1930 |
| Benzylglucopyranosid | 1 | 15 | 1785 |

### Beispiel 4

Der Einfluß verschiedener Detergens-Chargen auf den Eichkurvenverlauf und die Wiederfindung von Humanserum wurde am Beispiel Enzymun-Test ® LH ermittelt. Als Methode des Standes der Technik wurden verschiedene Pluronic ® F68-Chargen eingesetzt und die erhaltenen Ergebnisse mit verschiedenen Chargen von Benzylglucosid verglichen. Die Detergenzien wurden, wie in den vorhergehenden Beispielen, dem Inkubationspuffer des Enzymun-Test ® LH in einer Konzentration von 0,5 % zugesetzt. Die Wiederfindung wurde an verschiedenen Humanseren getestet.

Mit den jeweiligen Chargen wurden mit den Standards a - f Eichkurven erstellt. Aus den Extinktionen der Humanserumproben wurde über die Eichkurve die jeweilige IM-Konzentration abgelesen.

Aus Tabelle 3 ist zu erkennen, daß sich je nach verwendeter Pluronic F68-Charge die Eichkurven deutlich unterscheiden. Im Mittel differiert die Wiederfindung von Humanseren bei diesen Chargen um bis zu 16 %.

Aus Tabelle 4 ist zu erkennen, daß sich die Eichkurven bei Verwendung verschiedener Benzylglucosid-Chargen nicht signifikant unterscheiden. Die Wiederfindung bei Verwendung dieser Chargen differiert lediglich um 4 %.

**Tabelle 3**

| **Enzymun-Test ® LH** | | | | | | |
|---|---|---|---|---|---|---|
| **Pluronic F68-Chargenabhängigkeit** | | | | | | |
| **Einfluß auf Eichkurvenverlauf und Wiederfindung von Humanseren** | | | | | | |
| a) Eichkurven | | | | | | |
| Pluronic F68-Charge | A | | B | | C | |
| Standard a (mE) | 21 | | 17 | | 19 | |
| Standard b (mE) | 49 | | 44 | | 52 | |
| Standard c (mE) | 169 | | 1381 | | 1761 | |
| Standard d (mE) | 4111 | | 3411 | | 4341 | |
| Standard e (mE) | 10701 | | 8831 | | 11161 | |
| Standard f (mE) | 1832 | | 1536 | | 1925 | |

| b) Wiederfindung (WF) von Humanseren (HS) | | | | | | |
|---|---|---|---|---|---|---|
| | A | % WF | B | % WF | C | % WF |
| HS 1 (mIU/ml) | 15,9 | 100 | 17,2 | 108 | 14,7 | 92 |
| HS 2 (mIU/ml) | 8, 8 | 100 | 9, 1 | 103 | 7 , 5 | 85 |
| HS 3 (mIU/ml) | 9, 1 | 100 | 9, 4 | 103 | 7 , 9 | 87 |
| HS 4 (mIU/ml) | 6,3 | 100 | 6,9 | 110 | 5,5 | 87 |
| HS 5 (mIU/ml) | 64, 2 | 100 | 74, 9 | 117 | 65,0 | 101 |
| HS 6 (mIU/ml) | 48,7 | 100 | 56,0 | 115 | 48,7 | 100 |
| HS 7 (mIU/ml) | 48,1 | 100 | 51,5 | 107 | 43,7 | 91 |
| HS 8 (mIU/ml) | 35, 5 | 100 | 39,2 | 110 | 34,2 | 96 |
| mittl. Wiederfindung in % | | 100 | | 109 | | 93 |

**Tabelle 4**

| **Enzymun-Test ® LH** | | | | | | |
|---|---|---|---|---|---|---|
| **Einfluß verschiedener Chargen Benzylglucosid auf Eichkurvenverlauf und Wiederfindung von Humanseren** | | | | | | |
| a) Eichkurven | | | | | | |
| Benzylglucosid- Charge | A | | B | | C | |
| Standard a (mE) | 34 | | 33 | | 32 | |
| Standard b (mE) | 68 | | 68 | | 65 | |
| Standard c (mE) | 217 | | 220 | | 202 | |
| Standard d (mE) | 458 | | 466 | | 434 | |
| Standard e (mE) | 1166 | | 1176 | | 1131 | |
| Standard f (mE) | 2012 | | 2004 | | 1915 | |

| b) Wiederfindung (WF) von Humanseren (HS) | | | | | | |
|---|---|---|---|---|---|---|
| | A | % WF | B | % WF | C | % WF |
| HS 1 (mIU/ml) | 4,7 | 100 | 4,7 | 100 | 4,7 | 100 |
| HS 2 (mIU/ml) | 16,3 | 100 | 15,6 | 96 | 16,2 | 99 |
| HS 3 (mIU/ml) | 4,4 | 100 | 4,5 | 102 | 4,8 | 109 |
| HS 4 (mIU/ml) | 26,7 | 100 | 26,5 | 99 | 27,9 | 104 |
| mittl. Wieder findung in % | | 100 | | 99 | | 103 |

## Patentansprüche

1. Heterogenes Verfahren zurn immunologischen Nachweis eines Analyten, bei dem man den Analyten mit mindestens einem analytspezifischen Rezeptor zusammenbringt, der an eine Festphase gebunden ist oder gebunden werden kann, dadurch gekennzeichnet, daß man dem Inkubationsmedium ein wasserlösliches glykosidisches Tensid zusetzt.

2. Verfahren nach Anspruch 1, dadurch gekennzeichnet, daß als glykosidisches Tensid Hexyl-, Octyl- oder Benzylglucosid oder APGs auf der Basis von Hexyl-, Octyl- oder Benzylglucosid oder Gemische hiervon verwendet werden.

3. Verfahren nach Anspruch 1, dadurch gekennzeichnet, daß das Tensid in einer Konzentration von 0.1 - 2 % bezogen auf das Gewicht des gesamten Reaktionsansatzes eingesetzt wird.

4. Verwendung eines wasserlöslichen glykosidischen Tensids zur Vermeidung von unspezifischen Bindungen in immunologischen heterogenen Nachweisverfahren, bei denen mindestens ein spezifischer Rezeptor an eine Festphase gebunden ist oder gebunden werden kann.

5. Verwendung eines wasserlöslichen glykosidischen Tensids gemäß Anspruch 4, wobei als Probe Blutplasma eingesetzt wird.

## Revendications

1. Procédé hétérogène de détection immunologique d'un analyte, dans lequel on met en contact l'analyte avec au moins un récepteur spécifique à l'analyte qui est lié ou qui peut être lié à une phase fixe, caractérisé en ce qu'on ajoute au milieu d'incubation un surfactant glycosidique soluble dans l'eau.

2. Procédé selon la revendication 1, caractérisé en ce qu'on utilise, à titre de surfactant glycosidique, de l'hexyl-, de l'octyl- ou du benzylglucoside ou des APG à base d'hexyl-, d'octyl- ou de benzylglucoside ou des mélanges de ceux-ci.

3. Procédé selon la revendication 1, caractérisé en ce que le surfactant est utilisé en une concentration de 0,1 à 2 % par rapport au poids du mélange réactionnel total.

4. Utilisation d'un surfactant glycosidique soluble dans l'eau pour éviter des liaisons non-spécifiques dans des procédés hétérogènes de détection immunologique, dans lesquels au moins un récepteur spécifique est lié ou peut être lié à une phase fixe.

5. Utilisation d'un surfactant glycosidique soluble dans l'eau selon la revendication 4, où on utilise à titre d'échantillon du plasma sanguin.

## Claims

1. Heterogeneous method for the immunological detection of an analyte in which the analyte is brought into contact with at least one analyte-specific receptor which is bound to or can be bound to a solid phase, wherein a water-soluble glycosidic surfactant is added to the incubation medium.

2. Method as claimed in claim 1, wherein hexyl-glucoside, octylglucoside or benzylglucoside or APGs based on hexylglucoside, octylglucoside or benzylglucoside or mixtures thereof are used as the glycosidic surfactant.

3. Method as claimed in claim 1, wherein the surfactant is used at a concentration of 0.1 - 2 % in relation to the weight of the total reaction mixture.

4. Use of a water-soluble glycosidic surfactant to avoid unspecific binding in immunological heterogeneous detection methods in which at least one specific receptor is bound to or can be bound to a solid phase.

5. Use of a water-soluble glycosidic surfactant as claimed in claim 4, wherein blood plasma is used as the sample.
